# EUROPEAN PATENT SPECIFICATION

(11) **EP 0 127 681 B1**
(45) Date of publication and mention of the grant of the patent: **03.03.1993**
(21) Application number: 84900422.1
(22) Date of filing: 01.12.1983
(51) Int. Cl.: G01N 33/569, A61K 39/09

(54) **IgA BINDING PROTEIN**
Ig-A BINDENDES PROTEIN
PROTEINE LIANT D'IgA

(30) Priority: 02.12.1982 US 446317
(43) Date of publication of application: 12.12.1984
(73) Proprietor: THE ROCKEFELLER UNIVERSITY, New York, NY 10021 (US)
(72) Inventor: BLAKE, Milan, New York, NY 10021 (US); GOTSCHLICH, Emil, White Plains, NY 10603 (US); RUSSELL-JONES, Gregory, J., New York, NY 10021 (US)
(74) Representative: Weinhold, Peter, Dr.
(86) International application number: US8301904
(87) International publication number: WO8402194

(56) References cited:
- US-A- 4 271 147
- US-A- 4 413 057
- JOURNAL OF CLINICAL MICROBIOLOGY, vol. 13, no. 5, May 1981, pages 991-993; L. LINDAHL et al.: "New method that uses binding of immunoglobulin A to group A streptococcal immunoglobulin A Fc receptors for demonstration of microbial immunoglobulin A protease activity"
- INFECTION & IMMUNITY, vol. 26, no. 1, 1979, pages 143-149; M.KILIAN et al.: "Pathogenic species of the genus Haemophilus and Steptococcus pneumoniae produce immunoglobulin A1 protease"
- JOURNAL OF BIOCHEMICAL AND BIOPHYSICAL METHODS, vol. 2, 79-90 (1980), S. BHAKDI: "Removal of SDS from proteins for immunochemical analysis: A simple method utilizing ultracentrifugation in sucrose density gradients containing non-ionic detergent"
- JOURNAL OF EXPERIMENTAL MEDICINE, vol. 149, 1979, pages 58-66, The Rockefeller University Press; J.G. TAI et al.: "Isolation of type-specific polysaccharide antigen from group B type Ib streptococci"
- THE JOURNAL OF INFECTIOUS DISEASES, vol. 139, no. 1, January 1979, pages 89-92, The University of Chicago, M. BLAKE et al.: "Studies on Gonococcus infection. XVII.IgA1 cleaving protease in vaginal washings from women with gonorrhea"
- J. EXP. MED., vol. 152, November 1980, pages 1442-1447, The Rockefeller University Press, M.H. MULKS et al.: "IgA proteases of two distinct specificies are of two distinct specificies are released by Neisseria meningitidis"
- THE JOURNAL OF IMMUNOLOGY, vol. 109, no. 1, pages 90-96, 1972; H. RUSSELL et al.: "The isolation and some phyiochemical and biologic properties of the type III antigen of group B steptococci"

## Description

This invention is concerned with an IgA binding protein isolatable from the surface of a Group B streptococci, a product comprising said IgA binding protein absorbed on a solid support, a method of separating such IgA binding proteins and a method of detecting a pathogenic Neisseria meningitides or Neisseria gonorrhoeal infection.

IgA, as is known, is an immunoglobulin or class of antibody which is related to immunity against infections with bacteria and viruses at mucosal surfaces. It is present in all mammalian secretions including milk, spinal fluid and vaginal washings.

Like all human antibodies, IgA is comprised of heavy and light chains, and is characterized by a crystalline fraction, Fc, and an antibody fraction, Fab. There are two subclasses of IgA. These are IgA₁ and IgA₂. They differ in the structure of the heavy chains. Additionally, the antibodies exist in monomer form (molecular weight about 160,000), and dimer form in serum. Only the dimer form exists in mammalian secretions, and this is attached to an additional moiety known as the secretory piece. The IgA antibody, like all antibodies, is produced by the lymphocytes of the immune system. The secretory piece is produced by the epithelial cells lining the surface of body structures such as the breast, gut, salivary glands or genitourinary tract. The IgA produced by the lymphocytes is picked up by the epithelial cells from the side in contact with the circulating blood, the secretory piece is added, and the secretory IgA is secreted in the exterior surface of the epithelial cell.

In the course of combating bacterial and viral infections, IgA participates in the neutralization of antigens by routes which are known and understood. Many clinical tests for the presence of infection have been devised and are widely utilized which depend upon recognition of an interaction between IgA and antigens generated by the infection.

Group B streptococci is a class of microorganisms which has been extensively studied and classified. The publication of H. Russel et al. in The Journal of Immunology 109 (1), 90-96 (1972) refers to the isolation and some physiochemical and biologic properties of the type III antigen of Group B streptococci. The subject matter of this publication is principally the concept of isolating antigens to use for raising antibodies, either for the detection of a disease or for potential utility as a vaccine. It is concerned with carbohydrate antigens.

Furthermore from several publications it is known that many pathogenic bacteria, including certain Neisseria, Haemophilus and Streptococcus strains produce IgA proteases. However, there is no disclosure or any hint as to the presence of IgA binding proteins. On the contrary, no member of Group B streptococci has ever been known to bind to IgA.

It has now been discovered that certain of these streptococci, generally of the 1b or 1c serotype, will bind to IgA. Moreover, procedures have been discovered for separating an IgA binding protein (IgABP) from the surface of group B streptococci where it occurs.

One subject matter of this invention is a method of separating the IgA binding protein from the surface of a Group B streptococci which comprises extracting the streptococci in the log phase with an aqueous medium containing a material which will disrupt the bond between the cell surface and the protein to form a solution containing the protein and adding a protein precipitating reagent to precipitate the desired product.

One such procedure is to heat the streptococci in dilute aqueous mineral acid at 50°C to 60°C for from 1.5 to 2.5 hours. The preferred acid is dilute hydrochloric acid, e.g., 0.15 to 0.25 M, but other dilute mineral acids may be employed. The preferred procedure is to heat for two hours at 56°C in 0.2 M hydrochloric acid. The solution is neutralized with dilute alkaline reagent, e.g., 0.2M sodium hydroxide. The desired product may be precipitated by the addition of trichloroacetic acid or other reagent and then purified.

Another method of separating the IgA binding protein from the surface of a Group B streptococci comprises :
(a) extracting the streptococci in the log phase with an aqueous medium containing an anionic detergent,
(b) separating the solids portion of the resulting mixture,
(c) precipitating a solid phase from the resulting solution by the addition of a protein precipitating reagent.

As extraction medium preferably an aqueous puffer solution of 1% to 10% sodium dodecyl sulphate at pH 6 to 9 and as protein precipitation reagents preferably ethanol at 0°C to 10°C are used.

An alternative procedure comprises:
(a) extracting the streptococci in the log phase with an aqueous medium containing a non-ionic detergent,
(b) separating the solids portion of the resulting mixture,
(c) precipitating a solid phase from the resulting solution by the addition of a protein precipitating reagent.

According to this embodiment it is suitable to mix the streptococci in a dilute solution of the non-ionic detergent for a period of from about 36 to 72 hours, preferably 40 to 50 hours. The protein is precipitated with a selected reagent such as ethanol. It may be further purified.

The preferred procedure for isolating the IgABP of this invention is by boiling in an anionic detergent.

Suitable non-ionic detergents for use in this invention include Triton® X-100 and Tween® 20. Both are well known and available commercially. The former is a polyethylene glycol p-isooctylphenyl ether. The latter is polyoxyethylene sorbitan monooleate. The presently preferred anionic detergent is the commercially available sodium dodecyl sulfate. Others are known and can be used.

According to this embodiment the extraction medium may be an aqueous buffer solution of 1% to 10% polyethylene glycol p-isobutylphenyl ether at pH 6 to 9 and the protein precipitation may be carried out using ethanol at 0°C to 10°C.

The presently preferred procedure for isolating the IgABP of this invention using anionic detergent is as follows:
10 ml of the Group B streptococcal strain H36B 5 was grown to late-log phase in Todd-Hewitt broth. The bacteria were pelletted by centrifugation and then boiled in 1 ml of 2% SDS in water, for 10 minutes.

The bacteria were removed by centrifugation and the proteins in the supernatant were precipitated by the addition of 0.5 ml of 30% trichloroacetic acid. The pellet obtained by centrifugation was washed once with ethanol and once with acetone. The remaining pellet was suspended in 25 ml of the standard SDS-PAGE boiling solution and subjected to electrophoresis in a 10% polyacrylamide slab gel. After staining with Coomassie brilliant blue and destaining the SDS-PAGE pattern consisted predominantly of a single band with SDS-PAGE mobility corresponding to a protein of 132,000 molecular weight. A number of other minor bands with molecular weights ranging from 40,000 to 132,000 were also apparent.

A second preparation of the protein was also subjected to SDS-PAGE followed by western blotting onto nitrocellulose strips. The strip was then soaked in 0.05% Tween® 20 for 30 minutes, after which 1 x 10⁷ cpm of ¹²⁵I-labelled IgA at a concentration of 4 mg/ml was added to the strip for 4 hours. The strip was subsequently washed extensively with 0. 05% Tween® 20 and used to expose photographic film. By this technique it could be shown that the major protein band seen in the SDS-PAGE plus most of the minor proteins were also capable of binding the ¹²⁵I-IgA and thereby exposing the photographic film.

Molecular weight markers used in estimation of the molecular weight of the IgABP were as follows: β galactosidase (136kD), human transferrin (80kD), Ovalbumin (45kD), Cytochrome C (12.5kD).

SDS-PAGE is a well known and widely used process for separating proteinaceous materials using sodium dodecyl sulfate and polyacrylamide gel electrophoresis.

Once the desired material is separated from the streptococcus substrate it may be isolated and purified as described herein. Alternatively, solutions of the products in relatively dilute or impure states can be utilized.

Three procedures have thus far been described for isolating the useful products of this invention. In the last described process the product of the invention manifested a molecular weight of 132,000 on SDS-PAGE electrophoresis. This is a characteristic of the IgABP products of this invention. When subjected to SDS-PAGE electrophoresis the apparent molecular weight is 132,000.

If the products described above as precipitated with trichloroacetic acid or ethanol were boiled in 2% SDS and then treated as in the preferred procedure, there would be other major and minor bands when they were subjected to SDS-PAGE electrophoresis. These bands would be indicative of different molecular weights. However, they would be capable of binding IgA.

It is apparent therefore that the products of this invention, i.e., the IgA binding proteins, are characterized by a fixed fraction which binds the protein and a variable fraction which may be of different lengths, but always is associated with the binding portion. It is similar to a series of arrows, all of different lengths, but all useful for the same purpose since the shafts of differing length all carry an arrow head.

No IgA binding protein has ever been reported, much less isolated, from Group B streptococci.

The salient features of the novel IgABP of this invention can be employed in two distinct solid phase systems. In one such system the binding protein is absorbed on a solid phase support as in column chromatography. The second major application is to bind the IgABP to an absorbing surface such as a plastic plate, more specifically, a test well, and for use in a class of assays commonly known as the enzyme linked immunosorbent assay or ELISA.

The utilities for the first type of support are:
1. The IgA binding protein as a solid phase absorbent can be used to remove IgA of all classes from serum or any other human fluid. The eluate will be devoid of IgA. This is of value when one wishes to selectively remove antibodies of the IgA class from a secretion or serum. On the other hand, the IgA immunoglobulins can be removed from the solid phase support in highly purified form for any desired purpose.
2. The IgA binding protein as a solid phase absorbent can be used to determine the antigen specificity of IgA antibody. An antigen mixture which is labelled with radioactivity is exposed to the serum or secretion containing the IgA antibody. After antigen-antibody binding has been allowed to take place the solid support is added and binds only IgA antibody and any antigen that may be specifically bound to the IgA. The solid support is washed extensively and the quantity of antigen that is bound is identified by radioactivity. The nature of the bound antigen can be tested by performing a gel electrophoretic analysis followed by autoradiography or fluorography.

The application for the second type of support (plastic plate) are:
1. The IgA binding protein can be used to determine the concentration of IgA in serum or in a secretion. One strategy is to use a polystyrene or other plastic plate and absorb the IgA binding protein to the surface. After suitable washing, the plate is exposed to various dilutions of the fluid to be tested and after appropriate incubation is washed. The IgA bound is detected by an antibody specific for L chains (light chains) which has been conjugated to the enzyme alkaline phosphatase. The presence of this enzyme, and thus the L chains, is detected by release of the chromophore para-nitrophenol from para-nitrophenyl phosphate.
2. The IgA binding protein can be used as a detection system for the presence of gonorrhea. The principle is that the pathogenic gonococcus elaborates an enzyme capable of cleaving human IgA of the IgA₁ subclass in the middle of the molecule at a region known as the hinge region. This results in the prdouction of three fragments, the Fc portion of the molecule and two identical Fab fragments. The latter contain the L chains of the intact IgA₁ molecule. The test is carried out by coating a plastic plate with the IgA binding protein. The plate after washing is exposed to human IgA₁ immunoglobulin. After washing the secretion or vaginal washing is added to the wells and allowed to incubate. After washing the plate is exposed to alkaline phosphatase conjugated anti-light chain antibody and developed with the phosphatase substrate p-nitrophenyl phosphate. If the secretion contained active IgA protease then the Fab portions will have been cleaved from the molecule and the light chains lost from the well. A low color yield is thus an indication for the presence of the IgA₁ protease.
3. The IgA binding protein can be used to determine the presence of bacterial meningitis caused by Neisseria meningitidis, Haemophilus influenzae, and Diplococcus pneumoniae. All of these organisms produce a similar IgA₁ splitting protease. The test will be carried out exactly as described in section 2 except that the presence of the enzyme will be assayed in spinal fluid obtained by lumbar puncture which is performed routinely on patients suspected of having meningitis.
4. The IgA binding protein can be used to determine specific reactivity of antibodies of the IgA class. Since IgA is the major protective antibody found on the epithelial surface of the human body it is of major importance to be able to measure the IgA which is reactive with specific bacterial or viral antigens. This can be accomplished by using the IgA binding protein to coat the plates. The plates are allowed to bind. After washing a detection system for the presence of the antigen is used such as, for instance, an antibody to the bacterial product or virus in question, and this is coupled to an enzyme or radioactive detection system.
5. The IgA binding protein can be used to determine IgA antibodies specifically in each of the two subclasses, IgA₁ and IgA₂. The manner in which this is accomplished is that the same test outlined in item 4 is used except that before the specific antigen is added the IgA₁ antibodies fixed to the plate are destroyed with purified gonococcal or other IgA₁ protease. The difference is reactivity between test according to protocol 3 and 4 allows one to determine the contribution of antibodies in each subclass.

Those skilled in the art to which this invention pertains, once they have understood the procedures for isolating and identifying the IgABP of this invention as described herein, will readily understand how the products can be utilized in each of the procedures described above. They will, of course, recognize that it is not essential to isolate and purify the IgABP in order to practice the various embodiments of the invention. In fact, it will almost always be most convenient to utilize compositions containing the binding protein at various concentrations, for example the effluent from a chromatographic column containing the binding protein in solution. One such solution can be prepared by the following procedure.

A suspension of Group B streptococci is first tested to be certain that it will bind to IgA. This may be determined by incubating a pellet of streptococci with radiolabelled IgA at room temperature for about 20 minutes and then testing by conventional procedures for the presence of conjugated IgA. Specific known strains of Group B streptococci which are known and publically available and may be employed in the practice of this invention are A 909, F 345-3 and H 36B-5. The first two strains are 1c serotype. The last strain is 1b serotype.

A suspension of the selected strain in the log phase is extracted by boiling in 1% to 10% aqueous sodium decyl sulfate for 10 minutes to two hours at a pH of from about 6 to 9. The pH is not critical, but it is convenient to maintain it in this range with a suitable buffer such as 0.1M Tris-HC1. The bacteria are separated, for example by centrifugation. The protein remains in solution and may be precipitated by conventional procedures, including for example the addition of ammonium sulfate, ethanol, or other protein precipitating reagent.

It is most convenient to effect precipitation by the addition of ethanol at a temperature of about 0°C to 10°C to provide a mixture which is 60% to 90%, preferably 65% to 75% in ethanol.

The precipitated material is resuspended in water at a pH of from about 5.5 to 6.5 maintained by a suitable buffer such as glycine-hydrochloric acid, sodium acetate-acetic acid, or a citrate buffer. A 10 mM citrate buffer at pH 5.5 is most convenient. To remove the contaminating DNA or RNA, both of which are negatively charged, at this pH, the solution is passed over a DEAE-Sepharose® column which has been equilibrated with the same buffer employed in solubilizing the precipitate. The absorbent is positively charged and readily binds the DNA and RNA. The effluent from the column contains the binding protein in solution in sufficient concentration to be useful for the various purposes outlined above.

DEAE-Sepharose® is available from Pharmacia Fine Chemicals. It is a bead formed gel formed from agarose and converted to the diethylamino ethyl (DEAE) derivative. Other cationic exchange resins can be used.

As a further aid towards understanding this invention, the procedure for detecting a Neisseria gonorrhea or meningitides infection will now be described in more detail.

A constitutive extracellular enzyme, IgA₁ protease is released from the pathogenic Neisseria, N. meningitidis and gonorrhoeae respectively. This enzyme cleaves human IgA₁ into two fragments, the Fc fragment and the Fab fragment. An ELISA assay utilizing the IgABP isolated from group B streptococci has been developed in accordance with this invention which allows for the detection of the protease and, hence, for the presence of the Neisserial pathogens.

In this assay, the IgABP (bound to an ELISA plate) is used to bind the Fc portion of IgA. The IgA₁ protease can then cleave the IgA and release the Fab fragments which contain L-chains. The presence or absence of the light chains (corresponding to the absence or presence of the enzyme) can then be detected using a suitable reagent.

The assay is as follows:
1. IgABP ( 1-5 µg/ml in 0.1M Tris HC1 ph 9.8) is used to coat the surface of an ELISA plate with 100 µl/well at a pH from 6 to 11 at ambient temperature (20 to 40°C) for about 10 to 16 hours.
2. The plate is washed 6 times with a solution of 0.02 and 0.2% Brij® in 0.9% NaCl, i.e isotonic sodium chloride.
3. IgA (o.2 to 250 µg/ml in 10 mM Tris HC1 pH 7.5 plus 10 mM MgCl₂ 0.05% Brij® 35) is added to each well. The pH may be from 5 to 10. After one hour solutions containing the IgA₁ protease (viz: vaginal secretions) are added to the IgA and allowed to react for 15 minutes to 4 hours at ambient temperature.
4. The plate is washed as described above.
5. Alkalinephosphatase conjugated anti-human light chain sera is then added to each well and incubated at least 30 minutes and preferably 1 to 2 hours at ambient temperature.
6. The plate is washed as in 2.
7. The alkaline phosphatase substrate, para-nitrophenol phosphate suitably in a buffer at a pH of at least 7 and preferably 7 to 9 is added to each well and allowed to incubate for at least 30 minutes, suitably up to amost 2 hours at ambient temperature.
8. The OD₄₀₅ is then determined.

IgA₁ protease negative wells still contain intact IgA and so give a yellow color on reaction with the substrate. IgA₁ protease positive wells do not contain intact IgA, and so do not give a color reaction on addition of the substrate.

Brij® 35 is a non-ionic detergent comprising polyoxyethylene ethers of fatty acids. It is used to protect the absorbing surface from non-specific absorption of extraneous proteins. The magnesium chloride is not essential but is preferred, especially if phosphate buffers are employed, since the IgA₁ protease requires trace amounts of magnesium ions for optimum activity.

It will be apparent to those skilled in the art that at each step of the procedure variations can be made in such parameters as concentration, temperature and incubation time without adversely affecting the test. Equivalent buffers may be employed.

## Claims

1. An IgA binding protein isolatable from the surface of Group B streptococci, said protein having a molecular weight of 132,000 when determined by sodium dodecyl sulfate polyacrylamide gel electrophoresis and being capable of binding to human IgA by reacting specifically with the Fc portion thereof.

2. A method of separating the IgA binding protein according to claim 1 from the surface of a Group B streptococci which comprises extracting the streptococci in the log phase with an aqueous medium containing a material which will disrupt the bond between the cell surface and the protein to form a solution containing the protein and adding a protein precipitating reagent to precipitate the desired product.

3. A method according to claim 2 which comprises:
(a) extracting the streptococci in the log phase with an aqueous medium containing an anionic detergent,
(b) separating the solids portion of the resulting mixture,
(c) precipitating a solid phase from the resulting solution by the addition of a protein precipitating reagent.

4. A method according to claim 3 wherein the extraction medium is an aqueous buffer solution of 1% to 10% sodium dodecyl sulfate at pH 6 to 9 and the protein precipitation reagent is ethanol at 0°C to 10°C.

5. A method according to claim 2 which comprises:
(a) extracting the streptococci in the log phase with an aqueous medium containing a non-ionic detergent,
(b) separating the solids portion of the resulting mixture,
(c) precipitating a solid phase from the resulting solution by the addition of a protein precipitating reagent.

6. A method according to claim 5 wherein the extraction medium is an aqueous buffer solution of 1% to 10% polyethylene glycol p-isobutylphenyl ether at pH 6 to 9 and the protein precipitating reagent is ethanol at 0°C to 10°C.

7. A method according to claim 2 wherein the extraction medium is dilute aqueous hydrochloric acid.

8. A method of detecting a pathogenic Neisseria meningitidis or Neisseria gonorrhoea infection characterized by the presence of an IgA₁ protease in a body fluid which comprises the steps of:
(1) exposing an absorbing surface to a solution of IgA binding protein according to claim 1 at a pH of from 6 to 11 and ambient temperature to coat the absorbing surface with said binding protein;
(2) washing the surface with aqueous isotonic sodium chloride solution containing a non-ionic detergent;
(3) exposing the surface to an aqueous solution of IgA at a concentration of 0.2 to 250 µg/ml at pH 5 to 10 thereby to bind the IgA to the binding protein on the absorbing surface;
(4) incubating a secretory fluid from the mammal suspected of harboring the infection for 15 minutes to 4 hours at ambient temperature;
(5) washing the surface as in Step 2;
(6) incubating with alkaline phosphate conjugated anti-human light chain serum for at least 30 minutes at ambient temperature;
(7) washing the surface as in Step 2;
(8) incubating with para-nitrophenol phosphate in a buffer at a pH of at least 7 for from 30 minutes to 2 hours at ambient temperature, and
(9) determining the optical density of the resulting medium at 405 nm.

9. A method as in claim 8 wherein the solution of Step 3 contains magnesium chloride.

10. A product comprising an IgA binding protein according to claim 1 absorbed on a solid support.

11. A product as in claim 10 wherein the solid support is a solid phase for use in column chromatography or is a plastic plate.

## Patentansprüche

1. IgA-bindendes Protein, das von der Oberfläche von Gruppe-B-Streptokokken isolierbar ist und bei Bestimmung durch Natriumdodecylsulfat polyacrylamid-Gelelektrophorese ein Molekulargewicht von 132 000 hat und fähig ist, durch spezifische Reaktion mit dem Fc-Anteil von Human-IgA an letzteres zu binden.

2. Verfahren zur Abtrennung des IgA-bindenden Proteins gemäß Anspruch 1 von der Oberfläche von Gruppe-B-Streptokokken, das das Extrahieren der Streptokokken in der log-Phase mit einem wäßrigen Medium, das ein Material enthält, welches die Bindung zwischen der Zelloberfläche und dem Protein unter Bildung einer das Protein enthaltenden Lösung aufbricht, und die Zugabe eines proteinausfällenden Mittels zum Ausfällen des gewünschten Produktes umfaßt.

3. Verfahren nach Anspruch 2, das umfaßt:
(a) Extrahieren der Streptokokken in der log-Phase mit einem ein anionisches Detergenz enthaltenden wäßrigen Medium,
(b) Abtrennen des festen Anteils der erhaltenen Mischung,
(c) Ausfällen einer festen Phase aus der erhaltenen Lösung durch Zugabe eines proteinausfällenden Reagenzes.

4. Verfahren nach Anspruch 3, worin das Extraktionsmedium eine wäßrige Pufferlösung von 1 bis 10 % Natriumdodecylsulfat bei einem pH-Wert von 6 bis 9 und das Proteinausfällungsreagenz Ethanol bei 0 bis 10°C ist.

5. Verfahren nach Anspruch 2, das umfaßt:
(a) Extrahieren der Streptokokken in der log-Phase mit einem ein nicht-ionisches Detergenz enthaltenden wäßrigen Medium;
(b) Abtrennen des festen Anteils der erhaltenen Mischung,
(c) Ausfällen einer festen Phase aus der erhaltenen Lösung durch Zugabe eines proteinausfällenden Reagenzes.

6. Verfahren nach Anspruch 5, worin das Extraktionsmedium eine wäßrige Pufferlösung von 1 bis 10 % Polyethylenglykol-p-isobutylphenylether bei einem pH-Wert von 6 bis 9 und das proteinausfällende Reagenz Ethanol bei 0 bis 10°C ist.

7. Verfahren nach Anspruch 2, worin das Extraktionsmedium verdünnte wäßrige Salzsäure ist.

8. Verfahren zum Nachweis einer pathogenen Neisseria meningitidis oder Neisseria gonorrhoea, gekennzeichnet durch das Vorliegen einer IgA₁-Protease in einer Körperflüssigkeit, das die Schritte umfaßt:
(1) In-Kontakt-bringen einer absorbierenden Oberfläche mit einer Lösung eines IgA-bindenden Proteins gemäß Anspruch 1 bei einem pH-Wert von 6 bis 11 und Raumtemperatur zum Beschichten der absorbierenden Oberfläche mit dem bindenden Protein;
(2) Waschen der Oberfläche mit wäßriger isotonischer Natriumchloridlösung, die ein nicht-ionisches Detergenz enthält;
(3) In-Kontakt-bringen der Oberfläche mit der wäßrigen Lösung von IgA bei einer Konzentration von 0,2 bis 250 µg/ml bei einem pH-Wert von 5 bis 10, um dadurch das IgA an das bindende Protein auf der absorbierenden Oberfläche zu binden;
(4) Inkubieren einer Sekretflüssigkeit eines Menschen oder Säugetiers, bei dem eine Infektion vermutet wird, für 15 min bis 4 h bei Raumtemperatur;
(5) Waschen der Oberfläche wie in Schritt 2;
(6) Inkubieren mit an alkalische Phosphatase konjugiertem Anti-Human-Leichtkettenserum für mindestens 30 min bei Raumtemperatur;
(7) Waschen der Oberfläche wie in Schritt 2;
(8) Inkubieren mit para-Nitrophenolphosphat in einem Puffer bei einem pH-Wert von mindestens 7 für 30 min bis 2 h bei Raumtemperatur und
(9) Bestimmen der optischen Dichte des erhaltenen Mediums bei 405 nm.

9. Verfahren nach Anspruch 8, worin die Lösung von Schritt 3 Magnesiumchlorid enthält.

10. Produkt, das ein IgA-bindendes Protein gemäß Anspruch 1 auf einem festen Träger absorbiert enthält.

11. Produkt nach Anspruch 10, worin der feste Träger eine feste Phase zur Verwendung bei der Säulenchromatographie oder eine Kunststoffplatte ist.

## Revendications

1. Protéine de liaison d'IgA, isolable à partir de la surface de streptocoques du groupe B, ladite protéine ayant un poids moléculaire de 132 000, déterminé par électrophorèse sur gel de polyacrylamide au dodécylsulfate de sodium, et étant capable de se lier à IgA humaine en réagissant de façon spécifique avec la fraction cristalline (Fc) de celle-ci.

2. Procédé pour séparer la protéine de liaison d'IgA selon la revendication 1 de la surface de streptocoques du groupe B, qui comprend l'extraction des streptocoques dans la phase de croissance exponentielle par un milieu aqueux contenant une matière qui rompra la liaison entre la surface cellulaire et la protéine pour former une solution contenant la protéine, et l'addition d'un réactif de précipitation de protéine pour précipiter le produit souhaité.

3. Procédé selon la revendication 2, qui comprend :
(a) l'extraction des streptocoques dans la phase de croissance exponentielle par un milieu aqueux contenant un détergent anionique,
(b) la séparation de la portion de matières solides du mélange résultant,
(c) la précipitation d'une phase solide à partir de la solution résultante par l'addition d'un réactif de précipitation de protéine.

4. Procédé selon la revendication 3, dans lequel le milieu d'extraction est une solution tampon aqueuse de dodécylsulfate de sodium de 1 à 10 %, ayant un pH de 6 à 9, et le réactif de précipitation de protéine est de l'éthanol à 0 à 10°C.

5. Procédé selon la revendication 2, qui comprend :
(a) l'extraction des streptocoques dans la phase de croissance exponentielle par un milieu aqueux contenant un détergent non-ionique.
(b) la séparation de la portion de matières solides du mélange résultant,
(c) la précipitation d'une phase solide à partir de la solution résultante par l'addition d'un réactif de précipitation de protéine.

6. Procédé selon la revendication 5, dans lequel le milieu d'extraction est une solution tampon aqueuse de p-isobutylphényléther de polyéthylèneglycol de 1 à 10 %, ayant un pH de 6 à 9, et le réactif de précipitation de protéine est de l'éthanol de 0 à 10°C.

7. Procédé selon la revendication 2, dans lequel le milieu d'extraction est de l'acide chlorhydrique aqueux dilué.

8. Procédé de détection d'une infection pathogène Neisseria meningitidis ou Neisseria gonorrhoea, caractérisé par la présence d'une protéase d'IgA₁ dans un fluide corporel, qui comprend les étapes consistant à :
(1) exposer une surface absorbante à une solution de protéine de liaison d'IgA selon la revendication 1 à un pH de 6 à 11 et à la température ambiante pour enrober la surface absorbante avec ladite protéine de liaison ;
(2) laver la surface avec une solution aqueuse de chlorure de sodium isotonique contenant un détergent non-ionique ;
(3) exposer la surface à une solution aqueuse d'IgA à une concentration de 0,2 à 250 µg/ml à un pH de 5 à 10, en liant ainsi IgA à la protéine de liaison sur la surface absorbante ;
(4) incuber un fluide de sécrétion provenant d'un mammifère, que l'on suppose souffrir de l'infection, pendant 15 minutes à 4 heures à température ambiante ;
(5) laver la surface comme dans l'étape 2 ;
(6) incuber avec du sérum à chaîne légère anti-humain conjugué de phosphate alcalin pendant au moins 30 minutes à la température ambiante ;
(7) laver la surface comme dans l'étape 2 ;
(8) incuber avec du phosphate de para-nitrophénol dans un tampon à un pH d'au moins 7 pendant 30 minutes à 2 heures à la température ambiante, et
(9) déterminer la densité optique du milieu résultant à 405 nm.

9. Procédé selon la revendication 8, dans lequel la solution de l'étape 3 contient du chlorure de magnésium.

10. Produit comprenant une protéine de liaison d'IgA selon la revendication 1 absorbée sur un support solide.

11. Produit selon la revendication 10, dans lequel le support solide est une phase solide utilisable dans une colonne chromatographique ou est une plaque de matière plastique.
